Europäisches Patentamt

European Patent Office

Office européen des brevets

(19)

(11) **EP 1 408 447 A2**

(12) # DEMANDE DE BREVET EUROPEEN

(43) Date de publication:
**14.04.2004 Bulletin 2004/16**

(51) Int Cl.⁷: **G06T 5/00**, A61B 6/00

(21) Numéro de dépôt: **03102583.6**

(22) Date de dépôt: **18.08.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **20.08.2002 FR 0210422**

(71) Demandeur: **COMMISSARIAT A L'ENERGIE ATOMIQUE**
**75752 Paris Cédex 15 (FR)**

(72) Inventeurs:
• **Dinten, Jean-Marc**
**69008 Lyon (FR)**
• **Darboux, Michel**
**38000 Grenoble (FR)**

(74) Mandataire: **Poulin, Gérard et al**
**Société BREVATOME**
**3, rue du Docteur Lancereaux**
**75008 Paris (FR)**

(54) **Procédé d'estimation d'un rayonnement diffusé, notamment afin de corriger des mesures en tomographie ou ostédensitométrie**

(57) L'image d'un objet est améliorée en estimant le rayonnement diffusé qu'il transmet aux détecteurs. Pour cela, on se sert du rayonnement diffusé effectivement mesuré à travers un simulacre de l'objet, présentant des propriétés d'atténuation analogues, et qu'on modifie par des coefficients de pondération obtenus par une transformation des valeurs du rayonnement total reçu à travers l'objet (3) et le simulacre (8) sélectionné. On parvient ainsi à améliorer l'image sans faire subir une double irradiation à l'objet pour mesurer séparément le rayonnement diffusé. Les applications principales sont la tomographie, l'ostéodensitométrie et le contrôle non destructif.

Fig. 3

**Description**

Le sujet de cette invention est un procédé d'estimation d'un rayonnement diffusé, dont l'application principalement envisagée est la correction de mesures en tomographie ou ostéodensitométrie.

L'utilisation d'un rayonnement d'irradiation, présente l'inconvénient de produire un rayonnement diffusé important à travers l'objet examiné surtout dans le cas très fréquent d'un rayonnement divergent (conique ou en éventail). En d'autres termes, chacun des détecteurs situés derrière l'objet reçoit non seulement un rayonnement primaire, provenant directement de la source par un trajet rectiligne et ayant traversé une région bien définie de l'objet, mais un rayonnement diffusé de provenance indéterminée qui affecte la mesure et qu'il serait donc souhaitable de corriger.

Plusieurs procédés sont déjà pratiqués. C'est ainsi que le rayonnement primaire peut être mesuré seul si une collimation stricte des détecteurs et de la source est faite afin d'intercepter le rayonnement diffusé, mais ce procédé nécessite en pratique un balayage du faisceau qui est lent à accomplir, et pendant lequel on doit s'accommoder de mouvements du patient si on examine des êtres vivants.

On a aussi eu l'idée contraire de ne mesurer que le rayonnement diffusé. On dispose pour cela un réseau discontinu d'absorbeurs, comme des billes de plomb, entre l'objet et les détecteurs, pour arrêter localement le rayonnement primaire, de sorte que les détecteurs situés derrière ces absorbeurs ne mesurent que le rayonnement diffusé. Ce procédé appelé « beam stop » donne donc des tables ou nappes bidimensionnelles de valeur de rayonnement diffusé, qu'on complète par interpolation entre les détecteurs placés derrière les absorbeurs. Le rayonnement diffusé ainsi estimé est soustrait du rayonnement total mesuré séparément. Ce procédé est précis mais a l'inconvénient qu'il impose deux irradiations de l'objet et donc un doublement de la dose de rayons qu'il reçoit. Un dernier exemple de méthode de correction du rayonnement diffusé par des moyens matériels comporte l'emploi de grilles anti-diffusantes, mais leur efficacité n'est que partielle ; elle est insuffisante pour un faisceau conique, où le rayonnement diffusé peut être plusieurs fois supérieur au rayonnement primaire.

Enfin, il existe un certain nombre de méthodes numériques pour estimer le rayonnement diffusé, à partir de convolutions ou de déconvolutions des mesures par exemple ; on pourrait aussi citer le brevet français 2 759 800 pour un procédé numérique différent, analytique. Elles sont en général d'emploi délicat car elles dépendent de paramètres choisis par l'utilisateur (noyaux de convolution par exemple) qui ne donnent de bons résultats que dans des situations favorables, comme des petites zones où le rayonnement diffusé est faible, ou des objets au contenu relativement homogène. Il n'existe aucun procédé simple qui permette par exemple de corriger le rayonnement diffusé à travers le thorax ou d'autres grandes zones anatomiques, dont l'examen est fréquent mais qui sont défavorables pour corriger le rayonnement diffusé en raison de leur volume même et de l'hétérogénéité due à la présence d'une structure d'os complexe et dont la capacité d'atténuation du rayonnement est très différente de celle des tissus mous.

Mentionnons enfin le brevet américain 6 018 565 pour l'exposé d'une méthode mixte, à « beam stop » et convolution.

Un objet essentiel de l'invention est de proposer un procédé d'estimation et de correction de rayonnement diffusé qui puisse convenir pour des situations difficiles de contrôle non destructif d'objets inertes ou animés, ou tous procédés de reconstruction d'image.

Le procédé conforme à l'invention est, sous sa forme la plus générale, un procédé d'imagerie d'un objet par acquisitions multiples, comprenant une estimation d'un rayonnement diffusé provenant d'un rayonnement initial ayant traversé un objet en subissant une atténuation laissant passer un rayonnement total de mesure, caractérisé par :

- pour au moins une acquisition, une prise d'une table de mesures d'un rayonnement diffusé, obtenue en faisant passer le rayonnement initial par un simulacre de l'objet,
- et pour chacune des acquisitions, un calcul de coefficients de transposition entre le simulacre et l'objet, d'après le rayonnement initial, le rayonnement total de mesure à travers l'objet et un rayonnement total de mesure à travers le simulacre,
- et une pondération de la table de mesures avec les coefficients de transposition.

Avantageusement, le simulacre sera un bloc d'épaisseur constante et en une matière homogène, ayant une atténuation semblable à une matière de base de l'objet ; en général la prise de table de mesure sera une sélection dans une série de tables de mesures de rayonnement diffusé, obtenues auparavant en faisant successivement passer le rayonnement initial à travers une série respective de simulacres de l'objet, d'épaisseurs différentes mais constante ; et la sélection sera faite par comparaison d'une valeur du rayonnement total de mesure à travers l'objet et d'une valeur du rayonnement total de mesure à travers les simulacres.

Les coefficients de pondération sont généralement des rapports de valeurs d'une même fonctionnelle calculée pour l'objet et pour le simulacre. La fonctionnelle utilisée peut être égale au produit du rayonnement total de mesure par le logarithme du rapport de rayonnement total de mesure et du rayonnement initial.

L'invention sera maintenant décrite en référence aux figures, parmi lesquelles :

- la figure 1 est une vue générale d'une acquisition des mesures ;
- la figure 2 est une vue d'une acquisition de calibration ;
- et la figure 3 illustre les étapes du procédé.

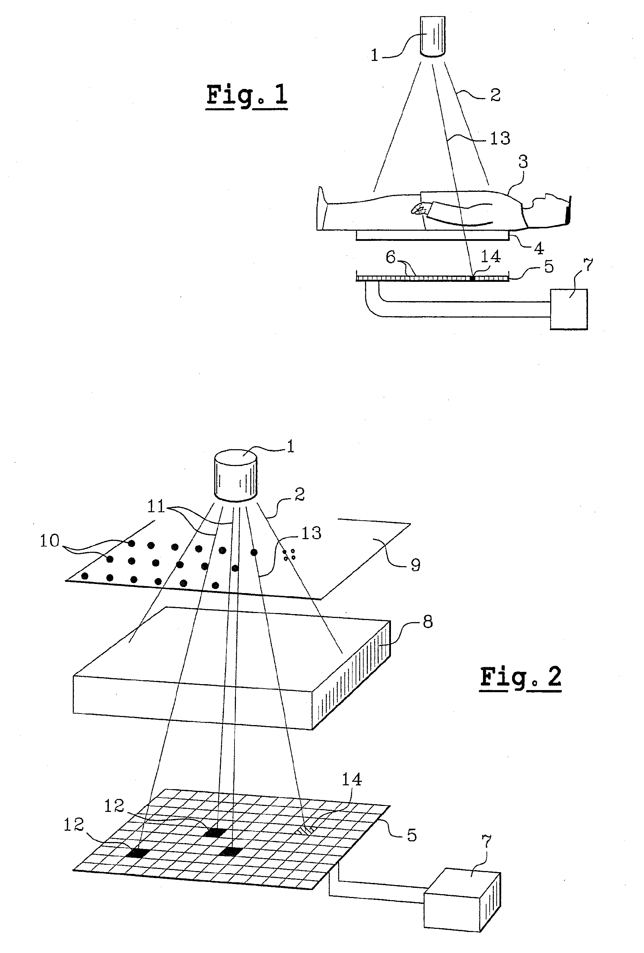

Reportons-nous d'abord à la figure 1, où un tube 1 de rayons X émet un faisceau 2 conique vers un objet 3 à examiner (ici un patient étendu sur une table 4) puis, à travers lui, vers un réseau 5 plan de détecteurs 6 disposés en matrice. Les détecteurs 6 sont reliés à un appareil d'acquisition 7 et mesurent un rayonnement diffusé qui se superpose au rayonnement primaire, seul convenable pour l'examen ou le contrôle de l'objet.

L'estimation du rayonnement diffusé à travers le patient (l'objet 3) consiste tout d'abord à obtenir des tables bidimensionnelles ou nappes de rayonnement diffusé obtenues dans des circonstances comparables. Pour cela, on effectue des irradiations d'étalonnage à travers des simulacres 8 de l'objet 3 à examiner, conformément à la figure 2 : les conditions d'irradiation restent les mêmes, c'est-à-dire qu'on continue d'utiliser le tube 1, le faisceau 2, le réseau 5 de détecteurs 6 et l'appareil d'acquisition 7, le simulacre 8 remplaçant cependant le patient ; on a aussi ajouté une grille 9 de billes 10 de plomb entre le simulacre 8 et le réseau 5. Il résulte de cette disposition que les rayons 11 passant par les billes 10 sont complètement absorbés et que les régions 12 du réseau 5 situées dans le prolongement de ces rayons 11 ont des détecteurs 6 qui ne mesurent que le rayonnement diffusé à ces endroits. Il suffit de relever ces valeurs mesurées et d'interpoler entre les régions 12 pour estimer convenablement le rayonnement diffusé issu du simulacre 8 pour tous les détecteurs 6 du réseau 5.

Le simulacre 8 devrait être semblable à l'objet afin que les rayonnements diffusés par eux fussent identiques. Une similitude parfaite n'est pas réalisable, et c'est pourquoi on se contente d'un simulacre 8 ressemblant à l'objet 3 et dont la nappe associée de rayonnement diffusé sera corrigée ultérieurement pour évaluer celle de l'objet. En pratique, le simulacre 8 peut être un bloc d'une matière homogène et qui présente le même coefficient d'atténuation que la matière de base de l'objet 3 : dans le cas d'un corps humain, composé pour l'essentiel de tissu mou, on sait que le plexiglas (polyméthacrylate) convient.

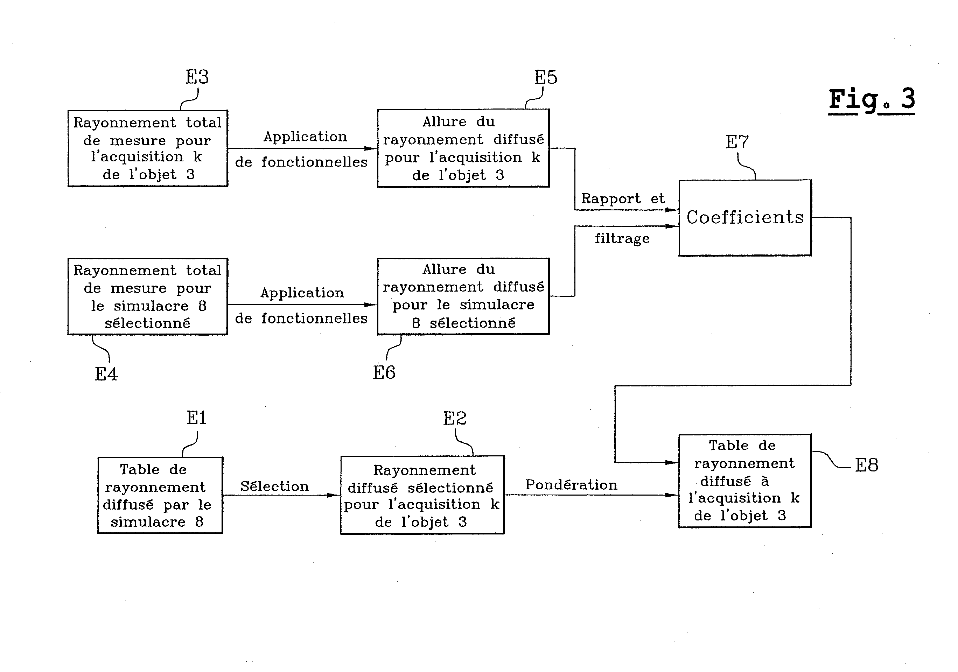

Afin de permettre des mesures variées, on disposera en réalité de plusieurs nappes de rayonnement diffusé, obtenues pour autant de simulacres 8, qui ne différeront que par leur épaisseur et donc par la longueur du trajet parcouru par les rayons 11. Ces nappes seront enregistrées dans une base de données préalablement aux mesures utiles sur les objets 3. pour prendre une nappe de rayonnement diffusé comparable à celle d'un objet 3, on sélectionnera en pratique une des nappes de la base de données ou, mieux, une nappe qu'on aura obtenue par des calculs d'interpolation entre deux de ces nappes. Le critère de sélection pourra être défini au moyen d'un rayon particulier 13 aboutissant à une région 14 du réseau 5 et qui ne passera ni par les absorbeurs 10 de la figure 2, ni par des tissus osseux du patient (ou plus généralement des portions de l'objet 3 dont les propriétés d'absorption sont différentes du matériau du simulacre 8) à la figure 1. Le rayonnement total, primaire et diffusé, reçu par la région 14 après avoir traversé chaque simulacre 8 servira d'index à la table de rayonnement diffusé correspondante, et la table sélectionnée aura l'index à une valeur identique au rayonnement total mesuré à la région 14 à travers l'objet 3. Tout cela correspond au passage de l'état E1 à l'état E2 dans l'organigramme de la figure 3, qu'on commence à commenter.

La suite du procédé consiste essentiellement en la correction de la table du rayonnement diffusé ainsi sélectionnée pour l'ajuster au mieux qu'on puisse espérer à la nappe de rayonnement réellement diffusé par l'objet 3. Pour cela, on se sert de toutes les informations disponibles, c'est-à-dire du rayonnement total reçu par les détecteurs 6 au-delà de l'objet 3 comme du simulacre 8 sélectionné. Ce rayonnement total étant noté $\Phi t$, le rayonnement diffusé $\Phi d$, le rayonnement initial issu du tube 1 $\Phi o$ et le rayonnement primaire $\Phi$, la relation $\Phi t = \Phi + \Phi d$ est respectée.

On est alors aux états E3 et E4 de l'organigramme de la figure 3. Ensuite, on transforme les valeurs des rayonnements totaux $\Phi t$ mesurées pour l'objet 3 et le simulacre 8 sélectionné en leur appliquant des fonctionnelles. Plus précisément, il est connu dans l'art que $\Phi d$ est proportionnel à $\Phi \log(\Phi/\Phi o)$ ; cette relation, qui est appelée la loi de Klein et Nishina, donne une allure générale du rayonnement diffusé, à défaut de son intensité.

Le rayonnement initial $\Phi 0$ est connu ; le rayonnement primaire $\Phi$ ne l'est pas, mais on consent à appliquer cette relation de façon approchée en le remplaçant par le rayonnement total $\Phi t$, c'est-à-dire que la fonctionnelle employée associe à chaque valeur mesurée du rayonnement total $\Phi t$ la valeur calculée $\Phi t \log(\Phi t/\Phi o)$, supposée proche du rayonnement diffusé $\Phi d$ à cet endroit ; on est parvenu aux états E5 et E6 de l'organigramme

L'étape suivante consiste à faire, pour chacun des détecteurs 6, le rapport des valeurs données par la fonctionnelle pour l'objet 3 et le simulacre 8 sélectionné selon la formule

$$K = \frac{\Phi t \log(\Phi t / \Phi o) \text{objet}}{\Phi t \log(\Phi t / \Phi o) \text{simulacre}}.$$

Les coefficients de pondération K ainsi obtenus serviront à déformer la nappe de rayonnement diffusé sélectionnée à l'état E2 afin d'estimer celle de l'objet 3. Les résultats constituent encore une table bidimensionnelle ou une matrice ayant des dimensions identiques à celle des tables de rayonnement puisqu'elle est associée au réseau 5 de détecteurs 6. Il est donc possible et avantageux d'effectuer un filtrage numérique spatial de cette matrice en appliquant un filtre passe-bas qui corrige les coefficients K en ne conservant que les fréquences les plus basses de leur variation et de les rendre ainsi probablement plus conformes à la réalité puisque le rayonnement diffusé varie assez lentement d'un point à un autre.

Quand la table des coefficients de pondération définitifs, notés K', a été obtenue (à l'état E7), elle sert à pondérer la table de rayonnement diffusé sélectionnée auparavant à l'état E2, pour obtenir une table de rayonnement diffusé par l'objet 3 (état E8, qui constitue l'estimation recherchée) ; la formule appliquée est Φd objet=K' Φd simulacre. Ces valeurs estimées Φd objet pourront alors être soustraites du rayonnement total Φt mesuré par les détecteurs 6 pour estimer le rayonnement primaire Φ et obtenir une image radiographique plus précise de l'objet 3.

Ce procédé s'applique aux irradiations à énergie simple ou multiple ; dans le second cas, il est répété séparément pour chacune des énergies employées.

La fonctionnelle proposée ici n'est pas la seule qu'on puisse employer, et la fonctionnelle plus simple Φd=kΦ (approchée ici encore en Φd=kΦt) , k étant une constante, pourrait aussi donner de bons résultats pour estimer Φd.

Ainsi qu'on l'a mentionné, ce procédé est particulièrement intéressant en tomographie ou ostéodensitométrie, où une image en profondeur de l'objet est reconstruite à partir d'une multitude d'acquisitions (irradiations) prises autour de l'objet par un réseau mobile de détecteurs, puis par une combinaison numérique de ces acquisitions (menée par des techniques qui ne font pas l'objet de l'invention). Une des limitations rencontrées en pratique est la dose excessive reçue par l'objet ; or on a vu que le procédé conforme à l'invention permettait de diminuer la dose nécessaire par rapport à d'autres, surtout ceux qui imposent une double irradiation pour soustraire le rayonnement diffusé du rayonnement total. On pressent que cet avantage sera sensible dans les procédés à réseau linéaire de détecteurs (ou à lignes superposées dans un réseau bidimensionnel où on obtient une ou plusieurs coupes (images bidimensionnelles) à travers l'objet.

Quand plusieurs acquisitions k sont entreprises, le mode opératoire résumé par la figure 3 peut être appliqué à chacune d'elles, une estimation indépendante du rayonnement diffusé étant faite à chaque fois. Il existe pourtant des situations où le rayonnement diffusé pourra être jugé invariable, notamment pour des objets à symétrie de révolution, très fréquente en procédés de contrôle non destructif. Plusieurs des étapes de la figure 3 deviendront alors inutiles : on pourra ainsi se contenter de la sélection d'un seul simulacre 8 pour toutes les acquisitions k ; éventuellement, seules les étapes E3, E5, E7 et E8 impliquant l'objet devront être répétées pour chaque acquisition. Le choix d'une simplification du procédé par l'omission de certaines calibrations pourra être à la discrétion de l'opérateur.

## Revendications

**1.** Procédé d'imagerie d'un objet par acquisitions multiples, comprenant une estimation d'un rayonnement diffusé provenant d'un rayonnement initial ayant traversé un objet (3) en subissant une atténuation laissant passer un rayonnement total de mesure, **caractérisé par** :

- pour au moins une des acquisitions, une prise d'une table de mesures d'un rayonnement diffusé, obtenue en faisant passer le rayonnement initial par un simulacre (8) de l'objet,
- et pour chacune des acquisitions, un calcul de coefficients (K') de transposition entre le simulacre et l'objet, d'après le rayonnement initial (Φo) , le rayonnement total de mesure à travers l'objet (Φt objet) et un rayonnement total de mesure à travers le simulacre (Φt simulacre),
- et une pondération de la table de mesures avec les coefficients de transposition.

**2.** Procédé d'imagerie selon la revendication 1, **caractérisé en ce que** le simulacre (8) est un bloc d'épaisseur constante et en une matière homogène, ayant une atténuation semblable à une matière de base de l'objet.

**3.** Procédé d'imagerie selon la revendication 1, **caractérisé en ce que** la prise de table de mesures est une sélection dans une série de tables de mesures de rayonnement diffusé, obtenues en faisant successivement passer le rayonnement initial à travers une série respective de simulacres de l'objet, qui sont des blocs d'épaisseurs différentes mais constante et en une matière homogène, ayant une atténuation semblable à une matière de base de l'objet.

**4.** Procédé d'imagerie selon la revendication 3, **caractérisé en ce que** la sélection comprend une interpolation entre deux des tables de mesures.

**5.** Procédé d'imagerie selon l'une quelconque des revendications 3 et 4, **caractérisé en ce que** la sélection est faite par comparaison d'une valeur du rayonnement total de mesure à travers l'objet et d'une valeur du rayonnement total de mesure à travers les simulacres.

**6.** Procédé d'imagerie selon la revendication 5, **caractérisé en ce que** la comparaison est faite pour des rayons identiques (13) du rayonnement initial à travers l'objet et les simulacres, ne traversant que la matière de base de l'objet.

**7.** Procédé d'imagerie selon l'une quelconque des revendications 1 à 6, **caractérisé en ce que** les coefficients de pondération sont des rapports de fonctionnelle identiques calculées pour l'objet et pour le simulacre.

**8.** Procédé d'imagerie selon la revendication 7, **caractérisé en ce que** les fonctionnelles sont égales au produit du rayonnement total de mesure par le logarithme du rapport du rayonnement total de mesure et du rayonnement initial.

**9.** Procédé d'imagerie selon l'une quelconque des revendications 1 à 8, **caractérisé en ce qu'**il comprend une étape de filtrage passe-bas des coefficients de transposition, arrangés en une table superposable à la table de mesures.

**10.** Procédé comprenant une étape de correction de mesures de radiographie par une soustraction d'un rayonnement diffusé estimé selon le procédé selon l'une quelconque des revendications précédentes.

**11.** Application du procédé selon l'une quelconque des revendications précédentes à la tomographie.

**12.** Application du procédé selon l'une quelconque des revendications 1 à 11 à l'ostéodensitométrie.

**13.** Application du procédé selon l'une quelconque des revendications 1 à 11 au contrôle non destructif.

Fig. 1

Fig. 2

**Fig. 3**

E3

| Rayonnement total de mesure pour l'acquisition k de l'objet 3 |

→ Application de fonctionnelles →

E5

| Allure du rayonnement diffusé pour l'acquisition k de l'objet 3 |

Rapport et filtrage →

E7

| Coefficients |

E4

| Rayonnement total de mesure pour le simulacre 8 sélectionné |

→ Application de fonctionnelles →

E6

| Allure du rayonnement diffusé pour le simulacre 8 sélectionné |

E1

| Table de rayonnement diffusé par le simulacre 8 |

→ Sélection →

E2

| Rayonnement diffusé sélectionné pour l'acquisition k de l'objet 3 |

→ Pondération →

| Table de rayonnement diffusé à l'acquisition k de l'objet 3 |

E8